# EUROPEAN PATENT SPECIFICATION

(11) **EP 0 427 051 B1**
(45) Date of publication and mention of the grant of the patent: **13.01.1999**
(21) Application number: 90120472.7
(22) Date of filing: 25.10.1990
(51) Int. Cl.: A61L 2/20, B65B 55/10

(54) **A method of producing a gaseous, hydrogen peroxide-containing sterilisation fluid**
Verfahren zur Produktion eines gasförmigen Fluidums, das Wasserstoffperoxyd enthält
Procédé de production d'un fluide gazeux contenant de l'hydrogène peroxyde

(30) Priority: 07.11.1989 SE 8903720
(43) Date of publication of application: 15.05.1991
(73) Proprietor: Tetra Laval Holdings & Finance SA, 1009 Pully (CH)
(72) Inventor: Andersson, Jan, S-271 00 Ystad (SE); Bjerborn, Thomas, S-222 27 Lund (SE); Martensson, Lars, S-240 14 Veberöd (SE); Smith, Göran, S-223 67 Lund (SE)
(74) Representative: Müller, Hans-Jürgen, Dipl.-Ing.

(56) References cited:
- EP-A- 0 321 908
- EP-A- 0 384 535
- GB-A- 2 183 480
- US-A- 4 797 255

## Description

The present invention relates to a method of producing a gaseous, hydrogen peroxide-containing sterilization fluid with good, uniform sterilization properties for the sterilization of packages and the like articles according to claim 1 and to a method of sterilizing material such as packaging material.

### BACKGROUND ART

Within packaging technology, use is often made of so-called aseptic packages for packing and transporting products which are particularly sensitive to bacterial attack and have short storage durability, for example foods and other perishable goods. The aseptic packages enjoy many advantages over other, non-aseptic packages: for example products of the above-mentioned sensitive type may be stored with retained or but insignificantly affected freshness for considerably longer storage times and, moreover, may be stored without the need for refrigeration or an unbroken refrigeration chain, which considerably increases and - in many respects - improves the distribution possibilities of the products involved. Fundamentally, the aseptic packaging technology or production of aseptic packages is based on the concept that a product which has been sterilised beforehand by heat treatment or other sterilisation methods is filled into a package or container likewise sterilised or produced from a sterilised packaging material, the container being thereafter sealed. The entire filling operation is carried out in a sterile environment in order to avoid reinfection of the sterilised product. Nowadays, such production is commonly carried out with the aid of modern, rational packaging machines of the type which, for example, both form, fill and seal the finished packages under the requisite aseptic conditions.

Using such a prior art packaging machine, single-use type aseptic packages are produced from prefabricated, creased-lined blanks of a laminated, flexible material, normally thermoplastic coated paper, with one or more additional layers of other materials than those mentioned here, in that the blank is first reformed into an open, tubular (normally square) carton which is thereafter given a liquid-tight bottom seal by folding and sealing together of end portions of the carton forming its bottom. After the bottom sealing, which most generally takes place in stages on a path along which the cartons are moved stepwise, the bottom-fitted cartons are introduced in the upright state into a sterilisation and filling zone aseptically screened-off from the ambient surroundings, for sterilisation and thereafter filling and sealing in a sterile atmosphere to form finished, aseptic packages for further distribution.

Using another prior art packaging machine, similar aseptic packages are produced in fundamentally the same manner as that described above, the only differences being that the tubular cartons are first provided with an injection moulded plastic seal serving as the top seal for the finished package at the one end of the carton, and that the cartons thus end-sealed are thereafter sterilised, filled and bottom-sealed in the "upside-down" position. One example of a package produced in accordance with the first-described production principle is the Tetra Rex (Registered Trademark), while one example of a package produced in accordance with the latter production principle is the Tetra Top (Registered Trademark).

Irrespective of whether the packages are produced according to the former or latter production principles, use is normally made of a gaseous, hydrogen peroxide-containing sterilisation fluid for sterilising the bottom-sealed or top-sealed package cartons ready for filling, since a gaseous sterilisation fluid may, in comparison with a corresponding liqueform fluid, more readily also penetrate into and sterilise concealed spaces of the cartons, for example folds formed as a result of the folding operation in the bottom seal of the cartons. At the same time, the gaseous fluid is moreover easier to ventilate off after completed sterilisation and prior to the filling operation. Furthermore, the gaseous sterilisation fluid enjoys that advantage that it wholly eliminates the risk of so-called edge-absorption in the cut edges of the cartons which show a ready tendency to suck up liquid and thereby render complete driving-off of hydrogen peroxide more difficult if not impossible. In order to preclude the risk of accompanying residual amounts of hydrogen peroxide arising out of such edge absorption, it is crucial that the gaseous sterilisation fluid be kept, throughout the entire sterilisation process, at a temperature which exceeds the dewpoint of the hydrogen peroxide, approximately 70°C, which implies that the package cartons must be heated to and kept at a temperature well above this dewpoint, normally approximately 80°C, on contact with the sterilisation fluid in the sterilisation zone.

An efficient hydrogen peroxide-containing sterilisation gas which is employed in sterilisation of packaging materials or package cartons for producing aseptic packages consists of an air/hydrogen peroxide mixture heated to approximately 120°C and containing approximately 25 g of hydrogen peroxide per 1 kg of air. Already after a very brief sterilisation time, of the order of 1 second, such a mixture gives a sterilisation result which fully satisfies the sterilisation requirements placed on aseptic packages.

A gaseous, hydrogen peroxide-containing sterilisation fluid of the above-considered type is produced, according to one prior art method, by spraying finely-divided, liqueform hydrogen peroxide onto a heated metal surface for vaporisation of the hydrogen peroxide, and then combining and mixing the vaporised hydrogen peroxide with a regulated, heated air current. This prior art method, which utilises heat transfer from a solid surface to the liqueform hydrogen peroxide, entails that the metal surface is gradually coated with impurities, for example stabilizers normally employed in liqueform hydrogen peroxide which, at high temperatures, act as catalysts for hydrogen peroxide degradation and contribute to a portion of the hydrogen peroxide being degraded and destroyed on vaporisation.

However, the problem inherent in "catalytic" degradation of hydrogen peroxide may be avoided by instead carrying out the vaporisation of the liqueform hydrogen peroxide using heated air as the heat transfer medium, and one prior art method which operates according to this vaporisation principle is based on the concept that the air intended for the vaporisation process is heated by heat exchange with a heating body heated electrically or by other means prior to the mixing operation with the liqueform hydrogen peroxide injected into the heated air current. This prior art method results in a gaseous hydrogen peroxide-containing sterilisation fluid with good sterilisation capability as long as it is carried out continuously, i.e. without any recurring interruptions. On the other hand, it has proved to function less satisfactorily - or defy problem-free execution - in those cases when, for one reason or another, it has been desirable to discontinue production from time to time for recurring downtimes of longer or shorter duration. As a rule, the sterilisation gas produced by intermittent vaporisation of the liqueform hydrogen peroxide has displayed extreme temperature variations and, hence, varying sterilisation capability, which has occasionally even been so poor that it has proved difficult or impossible to meet the requisite sterilisation standards. In order to ensure the desired sterilisation result in sterilisation of, for instance, package cartons which are to be filled with sterile contents, it has hitherto been a matter of necessity or expediency to produce the gaseous sterilisation fluid continuously throughout the entire sterilisation process, which has entailed that, for example, the fluid produced between two mutually subsequent carton sterilisations (and a priori to some extent superfluous) is wasted. By the same token, the fluid produced continuously during the ventilation of the sterilised cartons has also constituted an unneccessary excess production and an economic loss factor.

AU-B 33362/89 describes the sterilization of machine parts of a packaging machine which is not carried out during packaging. Cool air is heated in a heater. Then, the heated air is mixed with desinfectants being vaporized in a former step whereafter the mixture is lead through pipes to the machine parts which are to be sterilized. These pipes are preheated by superheated air wherein such heat is stored within these pipes in order to prevent the recondensation of the mixture on its way to the machine parts. Thereafter the mixture condenses on the machine parts to form a liquid film thereon.

### OBJECTS OF THE PRESENT INVENTION

One object of the present invention is, therefore, to propose a method of the type mentioned in the first part of claim 1 which method can also be carried out intermittently while retaining the good sterilisation properties of the intermittently produced fluid.

A further object of the present invention is to propose a method of the above-mentioned type which may readily be reduced into pratice on sterilisation of mutually subsequently conveyed package cartons or other objects without any part of the produced sterilisation fluid becoming superfluous and going to waste and without condensation on the walls of the packages.

Another object of the present invention is to propose a method of the type mentioned in the first part of claim 8 which method guarantees to satisfactorily clean the sterilized objects after the sterilization prozess by simple means.

### SOLUTION

The invention is characterized in claim 1 and 8 and preferred embodiments are claimed in subclaims.

According to the invention the vaporization agent is heated to and kept at a constant or substantially constant temperature above the vaporization temperature. The air current is brought into contact with heat exchange surfaces of a large thermal capacity and large mass, respectively.

Since the air current is, on each vaporisation occasion, kept at such a constant elevated vaporisation temperature (which has hitherto proved difficult to ensure without the use of a complex temperature and energy regulation apparatus), the requisite preconditions are created for the gaseous fluid produced throughout the entire production process to obtain a uniform or but insignificantly varying temperature and, coincidentally, the sought-for uniform and good sterilisation properties even if production is carried out with recurring brief or lengthy interruptions in the vaporisation of the liqueform hydrogen peroxide.

According to one particularly preferred embodiment of the method according to the present invention which may readily be reduced into practice, the vaporisation air is heated to and kept at the desired uniform vaporisation temperature in that it is caused to flow in direct contact with the heat transfer surface of a heating element of large mass (thermal capacity) and large heat exchange surface area which is heated electrically or by other means. The heating element may consist of aluminium or other material with superior thermal capacity which is capable of storing large amounts of thermal energy and, thus, does not require continuous energy supply to be kept sufficiently hot for the desired heating of the air current. Since, furthermore, at least a portion of the thermal energy stored in the heated heating element contributes in keeping the heat exchange surface of the heating element sufficiently hot for the desired heating of the air, it will be a simple matter to regulate the supply of thermal energy to the heating element.

### BRIEF DESCRIPTION OF THE ACCOMPANYING DRAWINGS

The nature of the present invention and its aspects will be more readily understood from the following brief description of the accompanying Drawings, and discussion relating thereto.

In the accompanying Drawings:

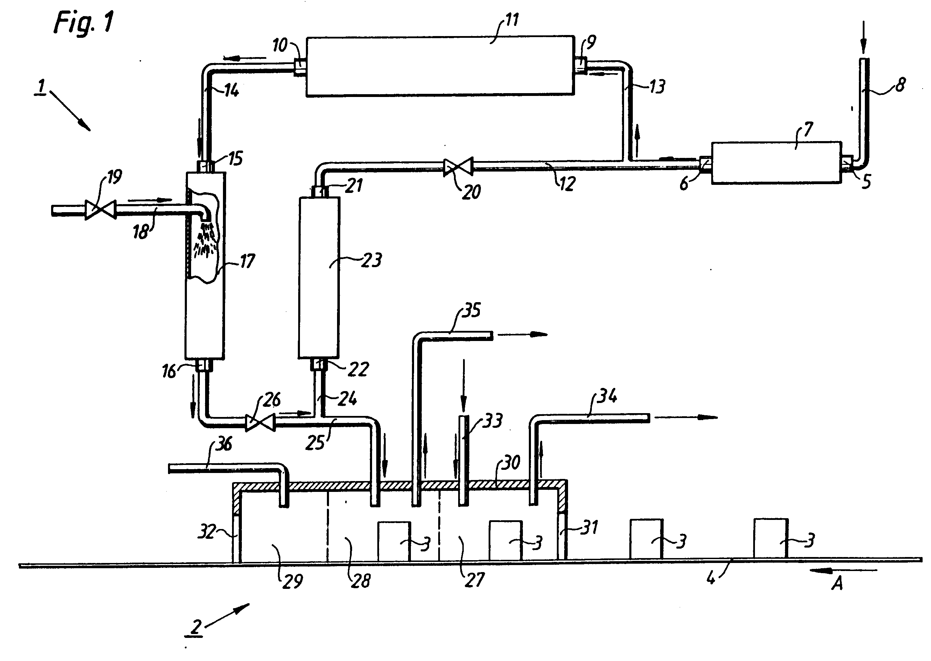

Figs. 1-3 schematically illustrate an apparatus for producing and using a gaseous, hydrogen peroxide-containing sterilisation fluid for sterilising objects intermittently conveyed in mutual sequence.

Since Figs. 1-3 illustrate the same apparatus, the apparatus will be described collectively with reference to all Figures at the same time, and - for purposes of clarity - the same reference numerals will be employed throughout for identical apparatus parts.

### DESCRIPTION OF PREFERRED EMBODIMENT

The system illustrated in Figs. 1-3 comprises a unit (carrying the generic reference numeral 1) for intermittent production of a gaseous, hydrogen peroxide-containing sterilisation fluid, and an apparatus (carrying the generic reference numeral 2) for sterilising objects 3 which are conveyed in mutual sequence on a conveyor belt 4 intermittently driven in the direction of the arrow A. The objects 3 may, for instance, be package cartons of the type described above and which are intended, after sterilisation, to be filled with sterile contents and thereafter sealed under aseptic conditions for the formation of so-called aseptic packages. While this description will hereinafter refer to the sterilisation of such package cartons, this should not be perceived as a restriction of the present invention which, in its broad scope, may naturally just as well be employed for the sterilisation of any other type of object which is to be sterilised under intermittent conditions.

The unit 1 includes a preheater 7 provided with an inlet 5 and an outlet 6, the preheater being in communication, via the inlet 5, with a conduit 8 for incoming filtered air. The preheater 7 is connected to a heat exchanger element 11 fitted with an inlet 9 and outlet 10, respectively, the inlet 9 being in communication, via a conduit 13, with a conduit 12 connected to the outlet 6 of the preheater 7. A conduit 14 leads from the outlet 10 of the heat exchanger element 11, the conduit 14 being connected to the inlet of a vaporisation chamber 17 fitted with an inlet 15 and outlet 16, a pipe 18 discharging into the vaporisation chamber for regulated supply (with the aid of a valve 19 in the pipe 18) of liqueform, preferably finely-divided hydrogen peroxide.

The conduit 12 connected to the outlet 6 of the preheater 7 is further connected, by the intermediary of an adjustable valve 20, to the inlet of a sterile filter unit 23 fitted with an inlet 21 and outlet 22. The sterile filter unit 23 is in communication, through a conduit 24 connected to the outlet 22, with a conduit 25 which in its turn is connected to the outlet 16 of the vaporisation chamber 17 and which is provided with an adjustable valve 26 between the outlet 16 of the vaporisation chamber and the communication with the conduit 24.

According to the present invention, the heat exchanger element 11 is of large mass (thermal capacity) and displays large heat exchange surface area and may, for instance, consist of aluminium or other material of superior thermal capacity with a capability of storing large volumes of thermal energy, as has been mentioned above. The heat exchange surface of the heat exchanger 11 is kept at the desired uniform, elevated temperature by regulated heating of its large mass, either electrically or by other means, for example by superheated steam in a per se known manner.

The apparatus 2 comprises a housing 30 divided up into discrete chambers or zones 27-29, the housing being provided with an inlet 31 and outlet 32 of the gate type for intermittent conveyance of the package cartons 3 stood on end on the conveyor belt 4 in sequence through the chambers or zones 27-29 in the housing 30.

The chamber 27 has an inflow pipe 33 and outflow pipe 34 for the throughflow of hot fluid, eg. air, for heating the package cartons 3, while the chamber 28 is connected to the conduit 25 of the unit 1 and is provided with an outflow pipe 35 for making possible a throughflow of fluids necessary for the sterilisation process.

The chamber 29 is provided with a filler pipe 36 discharging therein for filling sterile contents into the sterilised package cartons. A suitable device (not shown) is further provided in the chamber 29 for aseptic sealing of the filled package cartons prior to discharge through the gate outlet 32.

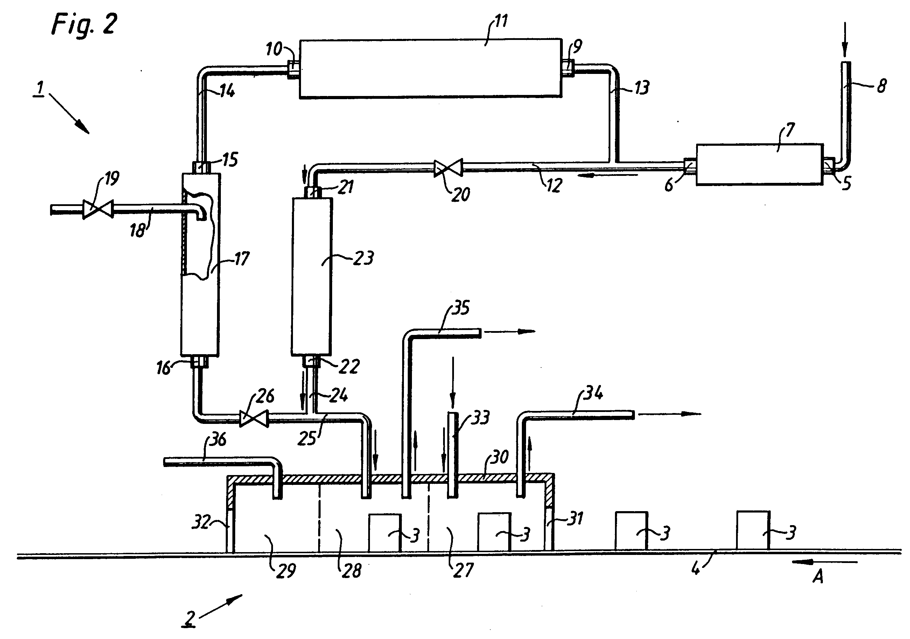

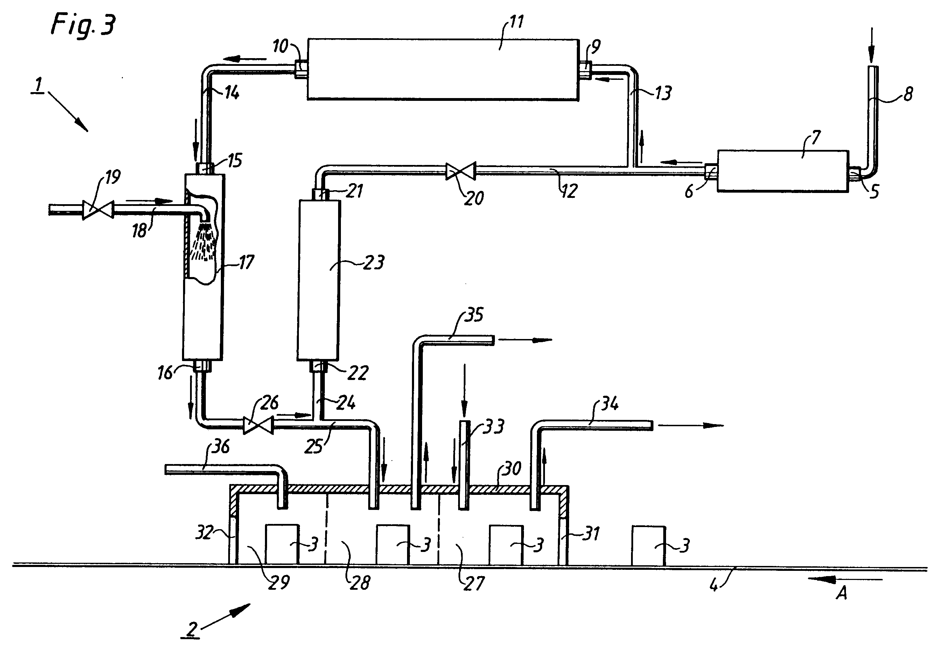

According to the present invention, the procedure is as follows for producing the gaseous, hydrogen peroxide-containing sterilisation fluid for sterilising the package cartons 3 employing the system described herein and shown on the Drawings. During the sterilisation (Fig. 1) of a package carton 3 fed into the sterilisation chamber 28, the valve 20 in the conduit 12 is closed, while the other valves included in the system, i.e. valves 19 and 26, are open. Filtered air incoming through the conduit 8 is preheated in the preheater 7 to approximately 90°C and is led via the conduit 13 into the heat exchanger element 11 where it is brought into contact with the heat exchange surface heated to approximately 400°C for heating to the desired elevated vaporisation temperature, of the order of 360°C. The thus heated air is withdrawn from the heat exchanger element 11 through the outlet 10 and is led through the conduit 14 into the vaporisation chamber 17 for intermingling with and vaporisation of the liqueform hydrogen peroxide fed in finely-divided form through the conduit 18, for the formation of the finished gaseous, hydrogen peroxide-containing sterilisation fluid at the desired uniform or but insignificantly varying temperature and thereby possessing good, uniform sterilisation properties. The thus obtained sterilisation fluid, which is at a temperature of approximately 120°C and has a hydrogen peroxide content corresponding to approximately 25 g of hydrogen peroxide per kg of supplied air, is withdrawn from the vaporisation chamber 17 and led via the conduit 25 into the sterilisation chamber 28 for sterilisation of the above-mentioned package carton 3. Spent sterilisation fluid is continuously removed from the chamber 28 through the outflow pipe 35 throughout the entire sterilisation period. After completed sterilisation (Fig. 2), which takes roughly 1 second, the supply of liqueform hydrogen peroxide is arrested, in that the valve 19 in the conduit 18 is closed, at the same time as the communication between the vaporisation chamber 17 and the sterilisation chamber 28 is broken by closure of the valve 26 in the conduit 25. The valve 20 in the conduit 12 is opened, whereby the air heated to approximately 90°C in the preheater 7 is allowed into the sterile filter unit 23 in which it is caused to pass through a sterile filter of per se known type for separation of any micro-organisms which may possibly be present in the air. The filtered air is withdrawn from the sterile filter unit 23 through the conduit 24 and is introduced, via the conduit 25, into the chamber 28 for ventilation and driving-off of residual sterilisation fluid after the sterilisation process proper, through the outflow pipe 35. The ventilation continues for approximately 0.5 seconds, whereafter the sterilised and ventilated package carton 3 is displaced by means of the conveyor belt 4 into the chamber 29 for filling with sterile contents through the filler pipe 36 (Fig. 3). During this displacement, the immediately subsequent package carton 3 preheated in the chamber 27 is conveyed simultaneously into the sterilisation chamber 28, the above-described sterilisation and ventilation cycle being repeated in that the valve 20 in the conduit 12 is closed and the valves 19 and 26 in the conduit 18 and the conduit 25, respectively, are opened for the supply of gaseous sterilisation fluid, freshly produced in the vaporisation chamber 17, to the chamber 28. The procedure is thereafter accordingly repeated in sequence for the remaining following package cartons on the conveyor belt 4 which conveys the cartons through the housing 30 in synchronised step with the above-mentioned sterilisation and ventilation in the chamber 28 and subsequent aseptic filling and sealing in the chamber 29 for the formation of finished aseptic packages progressively discharged through the gate outlet 32.

Given that the heat exchanger element 11 has large thermal capacity and large mass, respectively, the energy supplied for heating the heat exchange surface of the heat exchanger element may be discontinued or regulated during the above-described ventilation period without any risk of undesirable cooling of the heat exchange surface or excessive heating of the air waiting in the heat exchanger element, since the heat exchange surface will, during the discontinued or regulated supply of energy, be reliably maintained at the desired even temperature by heating from the heat stored in the heat exchanger element. It will thereby be ensured in the method according to the present invention that the air heated in the heat exchanger element is kept at a constant or in any event insignificantly varying vaporisation temperature when it departs from the heat exchanger element and is fed into the vaporisation chamber 17, even if production of the gaseous, hydrogen peroxide-containing sterilisation fluid were to be carried out with very frequently recurring interruptions, be they brief or lengthy, in the supply of the liqueform hydrogen peroxide.

Thus, according to the present invention it is possible, employing simple, easily regulated equipment, to produce a gaseous, hydrogen peroxide-containing sterilisation fluid at an ensured uniform or but insignificantly varying temperature, and with coincidentally stable, good sterilisation properties for efficient sterilisation of package cartons or other intermittently conveyed objects, without any part of the produced fluid needing to go to waste.

## Claims

1. A method of producing a gaseous, hydrogen peroxide-containing sterilisation fluid with good uniform sterilisation properties for the sterilization of packages and the like articles, carried out intermittently, by intermittent vaporization of liqueform hydrogen peroxide in a heated air current serving as a vaporization agent,
**characterized in that**
said air current is heated to and kept at a substantially constant temperature above the vaporization temperature by bringing said air current into contact with the heat transfer surface of a heating element (11) of large mass or thermal capacity and large heat exchange surface prior to the vaporization of the liqueform hydrogen peroxide.

2. A method as claimed in Claim 1,
**characterized in that**
said air current is caused to flow in direct contact with the surface of the heating element (11).

3. A method as claimed in Claim 1 or 2,
**characterized in that**
said air current is preheated by a preheater (7) prior to the heating of said heating element (11).

4. A method as claimed in one of the preceding claims,
**characterized in that**
the heating element (11), comprising said heat exchange surfaces, is kept at a constant or substantially constant elevated temperature by regulated heat supply by electric or other means.

5. A method as claimed in claim 4,
**characterized in that**
superheated steam is used for supplying the heat.

6. A method as claimed in one of the preceding claims,
**characterized in that**
said air current consists of air continuously aspirated in from the ambient atmosphere, is filtered and preheated to approximately 90°C by said preheater (7) and is thereafter heated to the desired vaporization temperature of approximately 360°C by said heating element (11).

7. A method as claimed in one of the preceding claims,
**characterized in that**
the gaseous, hydrogen peroxide containing sterilization fluid is formed by leading said heated air current through a vaporization chamber (17) and by injecting said liqueform hydrogen peroxide into said vaporization chamber (17) for intermingling with and vaporization of the liqueform hydrogen peroxide.

8. A method as claimed in claim 7,
**characterized in that**
the liqueform hydrogen peroxide is injected in finely-divided form into the heated air current and the injection of the liqueform hydrogen peroxide is discontinued during the vaporization interruptions.

9. A method for sterilizing material such as packaging material by producing said gaseous, hydrogen peroxide containing sterilization fluid as claimed in one of the preceding claims,
**characterized by**
introducing said gaseous, hydrogen peroxide containing sterilization fluid in a desinfection chamber (28) for sterilizing said material which is intermittently conveyed through said desinfection chamber (28).

10. A method as claimed in claim 9,
**characterized in that**
said air current preheated by said preheater (7) is led through a sterile filter unit (23) and into said desinfection chamber (28) for ventilation and driving-off residual sterilization fluid after the sterilization process.

11. A method as claimed in claim 9 or 10,
**characterized in that**
said sterile filter unit (23) with its supply conduit (12) and outflow conduit (24) lies in parallel to said heating element (11) and said vaporization chamber (17).

## Patentansprüche

1. Verfahren zur Herstellung eines gasförmigen, Wasserstoffperoxid enthaltenden Sterilisierungsfluids mit guten, gleichmäßigen Sterilisierungseigenschaften zur Sterilisierung von Packungen und ähnlichen Gegenständen, die intermittierend durch intermittierende Verdampfung von flüssigem Wasserstoffperoxid in einem erwärmten Luftstrom durchgeführt wird, der als Verdampfungsmittel dient,
**dadurch gekennzeichnet, daß**
der Luftstrom auf eine im wesentlichen konstante Temperatur über der Verdampfungstemperatur erwärmt und dort gehalten wird, indem der Luftstrom vor der Verdampfung des flüssigen Wasserstoffperoxids mit der Wärmeübertragungsfläche eines Heizelements (11) mit großer Masse oder Wärmekapazität und großer Wärmetauscherfläche in Kontakt gebracht wird.

2. Verfahren nach Anspruch 1,
**dadurch gekennzeichnet, daß**
man den Luftstrom in direktem Kontakt mit dem Heizelement (11) strömen läßt.

3. Verfahren nach Anspruch 1 oder 2,
**dadurch gekennzeichnet, daß**
der Luftstrom vor dem Erwärmen des Heizelements (11) von einer Vorwärmeinrichtung (7) vorgewärmt wird.

4. Verfahren nach einem der vorhergehenden Ansprüche,
**dadurch gekennzeichnet, daß**
das Heizelement (11) mit den Wärmetauscherflächen durch eine geregelte Wärmeversorgung durch elektrische oder andere Mittel auf einer konstanten oder im wesentlichen konstanten, hohen Temperatur gehalten wird.

5. Verfahren nach Anspruch 4,
**dadurch gekennzeichnet, daß**
überhitzter Dampf zur Wärmeversorgung verwendet wird.

6. Verfahren nach einem der vorhergehenden Ansprüche,
**dadurch gekennzeichnet, daß**
der Luftstrom aus Luft besteht, die kontinuierlich aus der Umgebungstemperatur angesaugt wird, gefiltert und von der Vorwärmeinrichtung (7) auf etwa 90°C vorgewärmt wird und danach von dem Heizelement (11) auf die gewünschte Verdampfungstemperatur von etwa 360°C erwärmt wird.

7. Verfahren nach einem der vorhergehenden Ansprüche,
**dadurch gekennzeichnet, daß**
das gasförmige, Wasserstoffperoxid enthaltende Sterilisierungsfluid gebildet wird, indem der erwärmte Luftstrom durch eine Verdampfungskammer (17) geführt und das flüssige Waserstoffperoxid zum Mischen damit und zum Verdampfen des flüssigen Wasserstoffperoxids in die Verdampfungskammer (17) eingespritzt wird.

8. Verfahren nach Anspruch 7,
**dadurch gekennzeichnet, daß**
das flüssige Wasserstoffperoxid in feinverteilter Form in den erwärmten Lufstrom eingespritzt und das Einspritzen des flüssigen Wasserstoffperoxids während der Verdampfungsunterbrechungen abgebrochen wird.

9. Verfahren zum Sterilisieren eines Materials wie eines Verpackungsmaterials durch Herstellung des gasförmigen, Wasserstoffperoxid enthaltenden Sterilisierungsfluids nach einem der vorhergehenden Ansprüche,
**dadurch gekennzeichnet, daß**
das gasförmige, Wasserstoff enthaltende Sterilisierungsfluid in eine Desinfektionskammer (28) eingebracht wird, um das Material zu sterilisieren, das intermittierend durch die Desinfektionskammer (28) befördert wird.

10. Verfahren nach Anspruch 9,
**dadurch gekennzeichnet, daß**
der von der Vorwärmeinrichtung (7) vorgewärmte Luftstrom zum Entlüften und Austreiben von Reststerilisierungsfluid nach dem Sterilisierungsvorgang durch eine sterile Filtereinheit (23) und in die Desinfektionskammer (28) geführt wird.

11. Verfahren nach Anspruch 9 oder 10,
**dadurch gekennzeichnet, daß**
die sterile Filtereinheit (23) mit ihrer Versorgungsleitung (12) und der Abflußleitung (24) parallel zu dem Heizelement (11) und der Verdampfungskammer (17) liegt.

## Revendications

1. Procédé de production d'un fluide de stérilisation gazeux contenant de l'hydrogène peroxyde ayant des propriétés de stérilisation bonnes et uniformes pour la stérilisation d'emballages et d'articles analogues, effectuée de façon intermittente, par vaporisation intermittente d'hydrogène peroxyde sous forme liquide dans un courant d'air chauffé servant d'agent de vaporisation, caractérisé en ce que ledit courant d'air est chauffé et maintenu à une température sensiblement constante supérieure à température de vaporisation par la mise en contact dudit courant d'air avec la surface de transfert de chaleur d'un élément chauffant (11) de grande masse ou de grande capacité thermique et de grande surface d'échange de chaleur préalablement à la vaporisation de l'hydrogène peroxyde sous forme liquide.

2. Procédé selon la revendication 1, caractérisé en ce que ledit courant d'air circule en contact direct avec la surface de l'élément chauffant (11).

3. Procédé selon la revendication 1 ou 2, caractérisé en ce que ledit courant d'air est préchauffé par un dispositif de préchauffage (7) avant le chauffage par ledit élément chauffant (11).

4. Procédé selon l'une des revendications précédentes, caractérisé en ce que l'élément chauffant (11) comprenant lesdites surfaces d'échange de chaleur, est maintenu à une température élevée constante ou sensiblement constante par fourniture régulée de chaleur par un moyen électrique ou autre.

5. Procédé selon la revendication 4 , caractérisé en ce que l'on utilise de la vapeur surchauffée pour fournir la chaleur.

6. Procédé selon l'une des revendications précédentes, caractérisé en ce que ledit courant d'air consiste en de l'air aspiré de façon continue à partir de l'atmosphère ambiante, est filtré et préchauffé jusqu'à environ 90°C par ledit dispositif de préchauffage (7) et est chauffé après cela à la température de vaporisation désirée d'environ 360°C par ledit élément chauffant (11).

7. Procédé selon l'une des revendications précédentes, caractérisé en ce que le fluide de stérilisation gazeux contenant de l'hydrogène peroxyde est formé en menant ledit courant d'air chauffé à travers une chambre de vaporisation (17), et en injectant dudit hydrogène peroxyde sous forme liquide dans ladite chambre de vaporisation (17) pour se mélanger avec lui et vaporiser l'hydrogène peroxyde sous forme liquide.

8. Procédé selon la revendication 7, caractérisé en ce que l'hydrogène peroxyde sous forme liquide est injecté sous forme finement divisée dans le courant d'air chauffé et l'injection de l'hydrogène peroxyde sous forme liquide est discontinue pendant les interruptions de vaporisation.

9. Procédé pour stériliser un matériau tel qu'un matériau d'emballage par production dudit fluide de stérilisation gazeux contenant de l'hydrogène peroxyde selon l'une des revendications précédentes, caractérisé par l'introduction dudit fluide de stérilisation gazeux contenant de l'hydrogène peroxyde dans une chambre de désinfection (28) pour stériliser ledit matériau qui traverse de façon intermittente la chambre de désinfection (28).

10. Procédé selon la revendication 9, caractérisé en ce que le dit courant d'air préchauffé par ledit dispositif de préchauffage (7) traverse un ensemble de filtre stérile (23) et pénètre dans ladite chambre de désinfection (28) pour ventiler et évacuer le fluide de stérilisation résiduel après le processus de stérilisation.

11. Procédé selon la revendication 9 ou 10, caractérisé en ce que ledit ensemble de filtre stérile (23) avec son conduit d'alimentation (12) et son conduit de sortie (24) est disposé en parallèle avec ledit élément chauffant (11) et à la dite chambre de vaporisation (17).
